# EUROPEAN PATENT APPLICATION

(11) **EP 2 263 696 A1**
(43) Date of publication of application: **22.12.2010**
(21) Application number: 10168156.7
(22) Date of filing: 02.04.2007
(51) Int. Cl.: A61K 47/48, C12N 15/62, C12N 9/64

(54) **Method of increasing the in vivo recovery of therapeutic polypeptides**

(30) Priority: 11.04.2006 EP 06007552
(62) Divisional of application: 07723889.7
(71) Applicant: CSL Behring GmbH, 35041 Marburg (DE)
(72) Inventor: Weimer, Thomas, Gladenbach, 35075 (DE); Metzner, Hubert, 35041, Marburg (DE); Schulte, Stefan, 35043, Marburg (DE); Lang, Wiegand, 35091, Cölbe (DE); Wormsbächer, Wilfried, 35274, Kirchhain (DE)
(74) Representative: Hauser, Hans-Peter

(57) **Abstract**

The present invention relates to the field of modified therapeutic polypeptides with increased in vivo recovery compared to their non-modified parent polypeptide. I.e., the invention relates to fusions of therapeutic polypeptides with recovery enhancing polypeptides connected directly or optionally connected by a linker peptide.

SEQUENCE LISTING

<110>  CSL Behring GmbH

<120>  Method of increasing the in vivo recovery of therapeutic proteins

<130>  2006.M003-A114-EP

<160>  38

<170>  PatentIn version 3.3

<210>  1
<211>  19
<212>  DNA
<213>  Artificial

<220>
<223>  PCR primer

<400>  1
ggcaggggca gcactgcag                                                    19


<210>  2
<211>  19
<212>  DNA
<213>  Artificial

<220>
<223>  PCR primer

<400>  2
cacaggccag ggctgctgg                                                    19


<210>  3
<211>  27
<212>  DNA
<213>  Artificial

<220>
<223>  PCR primer

<400>  3
gcggctagca tggtctccca ggccctc                                           27


<210>  4
<211>  30
<212>  DNA
<213>  Artificial

<220>
<223>  PCR primer

<400>  4
gcggcggccg cctagggaaa tggggctcgc                                        30


<210>  5
<211>  33
<212>  DNA
<213>  Artificial

<220>
<223>  Mutagenesis primer

<400>  5

gagccccatt tccctcgagg gccgccgcaa ggg                                33

<210>  6
<211>  33
<212>  DNA
<213>  Artificial

<220>
<223>  Mutagenesis primer

<400>  6
cccttgcggc ggccctcgag ggaaatgggg ctc                                33

<210>  7
<211>  61
<212>  DNA
<213>  Artificial

<220>
<223>  PCR primer

<400>  7
gtggtgctcg agcgtgcccc gcgccgtggg cggctccggc ggctccggcg gctccggatc    60

c                                                                   61

<210>  8
<211>  62
<212>  DNA
<213>  artificial

<220>
<223>  PCR primer

<400>  8
caccacgcgg ccgcttatca ggatccggag ccgccggagc cgccggagcc gcccacggcg    60

cg                                                                  62

<210>  9
<211>  52
<212>  DNA
<213>  artificial

<220>
<223>  PCR primer

<400>  9
ctcgagcggg ggatctggcg ggtctggagg ctctggaggg tcgggaggct ct            52

<210>  10
<211>  37
<212>  DNA
<213>  artificial

<220>
<223>  PCR primer

<400>  10
ggatccagag cctcccgacc ctccagagcc tccagac                            37

<210>  11
<211>  59

<212>  DNA
<213>  artificial

<220>
<223>  PCR primer

<400>  11
ggatcccgac cctccagacc cgccagatcc cccagagcct ccagagcctc ccgaccctc      59


<210>  12
<211>  49
<212>  DNA
<213>  artificial

<220>
<223>  PCR primer

<400>  12
ctcgagcaac ggatctggcg ggtctggagg ctctggaggg tcgggaggc                 49


<210>  13
<211>  48
<212>  DNA
<213>  artificial

<220>
<223>  PCR primer

<400>  13
ggatccgttt cccccagagc ctccagagcc tcccgaccct ccagagcc                  48


<210>  14
<211>  64
<212>  DNA
<213>  artificial

<220>
<223>  PCR primer

<400>  14
ggatccgttc cctccagacc cgccagatcc cccagagcct ccagagcctc ccgaccctcc     60

agag                                                                  64


<210>  15
<211>  56
<212>  DNA
<213>  artificial

<220>
<223>  PCR primer

<400>  15
ctcgagcaat ggatctggcg ggtctggagg ctctggaggg tcgaatggct ctggag         56


<210>  16
<211>  64
<212>  DNA
<213>  artificial

<220>
<223>  PCR primer

<400>  16

ggatccgttc cctccagacc cgccagatcc cccagagcct ccagagccat tcgaccctcc     60

agag                                                                  64


<210>  17
<211>  31
<212>  DNA
<213>  artificial

<220>
<223>  PCR primer

<400>  17
gtgggatccg atgcacacaa gagtgaggtt g                                    31


<210>  18
<211>  35
<212>  DNA
<213>  artificial

<220>
<223>  PCR primer

<400>  18
cacggatccc tataagccta aggcagcttg acttg                                35


<210>  19
<211>  406
<212>  PRT
<213>  human

<400>  19

<210>  20
<211>  585
<212>  PRT
<213>  human

<400>  20

<210>  21
<211>  415
<212>  PRT
<213>  human

<400>  21

Tyr Asn Ser Gly Lys Leu Glu Glu Phe Val Gln Gly Asn Leu Glu Arg
1               5                   10                  15

Glu Cys Met Glu Glu Lys Cys Ser Phe Glu Glu Ala Arg Glu Val Phe
            20                  25                  30

Glu Asn Thr Glu Arg Thr Thr Glu Phe Trp Lys Gln Tyr Val Asp Gly
        35                  40                  45

Asp Gln Cys Glu Ser Asn Pro Cys Leu Asn Gly Gly Ser Cys Lys Asp
    50                  55                  60

Asp Ile Asn Ser Tyr Glu Cys Trp Cys Pro Phe Gly Phe Glu Gly Lys
65                  70                  75                  80

Asn Cys Glu Leu Asp Val Thr Cys Asn Ile Lys Asn Gly Arg Cys Glu
                85                  90                  95

Gln Phe Cys Lys Asn Ser Ala Asp Asn Lys Val Val Cys Ser Cys Thr
            100                 105                 110

Glu Gly Tyr Arg Leu Ala Glu Asn Gln Lys Ser Cys Glu Pro Ala Val
        115                 120                 125

Pro Phe Pro Cys Gly Arg Val Ser Val Ser Gln Thr Ser Lys Leu Thr
    130                 135                 140

Arg Ala Glu Thr Val Phe Pro Asp Val Asp Tyr Val Asn Ser Thr Glu
145                 150                 155                 160

Ala Glu Thr Ile Leu Asp Asn Ile Thr Gln Ser Thr Gln Ser Phe Asn
                165                 170                 175

Asp Phe Thr Arg Val Val Gly Gly Glu Asp Ala Lys Pro Gly Gln Phe
            180                 185                 190

Pro Trp Gln Val Val Leu Asn Gly Lys Val Asp Ala Phe Cys Gly Gly
        195                 200                 205

Ser Ile Val Asn Glu Lys Trp Ile Val Thr Ala Ala His Cys Val Glu
    210                 215                 220

Thr Gly Val Lys Ile Thr Val Val Ala Gly Glu His Asn Ile Glu Glu
225                 230                 235                 240

Thr Glu His Thr Glu Gln Lys Arg Asn Val Ile Arg Ile Ile Pro His
                245                 250                 255

His Asn Tyr Asn Ala Ala Ile Asn Lys Tyr Asn His Asp Ile Ala Leu
            260                 265                 270

Leu Glu Leu Asp Glu Pro Leu Val Leu Asn Ser Tyr Val Thr Pro Ile
        275                 280                 285

Cys Ile Ala Asp Lys Glu Tyr Thr Asn Ile Phe Leu Lys Phe Gly Ser
    290                 295                 300

Gly Tyr Val Ser Gly Trp Gly Arg Val Phe His Lys Gly Arg Ser Ala
305                 310                 315                 320

Leu Val Leu Gln Tyr Leu Arg Val Pro Leu Val Asp Arg Ala Thr Cys
                325                 330                 335

Leu Arg Ser Thr Lys Phe Thr Ile Tyr Asn Asn Met Phe Cys Ala Gly
            340                 345                 350

Phe His Glu Gly Gly Arg Asp Ser Cys Gln Gly Asp Ser Gly Gly Pro
        355                 360                 365

His Val Thr Glu Val Glu Gly Thr Ser Phe Leu Thr Gly Ile Ile Ser
    370                 375                 380

Trp Gly Glu Glu Cys Ala Met Lys Gly Lys Tyr Gly Ile Tyr Thr Lys
385                 390                 395                 400

Val Ser Arg Tyr Val Asn Trp Ile Lys Glu Lys Thr Lys Leu Thr
                405                 410                 415

<210>  22
<211>  1335
<212>  DNA
<213>  human

<400>  22
atggtctccc aggccctcag gctcctctgc cttctgcttg ggcttcaggg ctgcctggct      60

gcagtcttcg taacccagga ggaagcccac ggcgtcctgc accggcgccg gcgcgccaac 120

gcgttcctgg aggagctgcg gccgggctcc ctggagaggg agtgcaagga ggagcagtgc 180

tccttcgagg aggcccggga gatcttcaag gacgcggaga ggacgaagct gttctggatt 240

tcttacagtg atggggacca gtgtgcctca agtccatgcc agaatggggg ctcctgcaag 300

gaccagctcc agtcctatat ctgcttctgc ctccctgcct tcgagggccg gaactgtgag 360

acgcacaagg atgaccagct gatctgtgtg aacgagaacg gcggctgtga gcagtactgc 420

agtgaccaca cgggcaccaa gcgctcctgt cggtgccacg aggggtactc tctgctggca 480

gacggggtgt cctgcacacc cacagttgaa tatccatgtg gaaaaatacc tattctagaa 540

aaaagaaatg ccagcaaacc ccaaggccga attgtggggg gcaaggtgtg ccccaaaggg 600

gagtgtccat ggcaggtcct gttgttggtg aatggagctc agttgtgtgg ggggaccctg 660

atcaacacca tctgggtggt ctccgcggcc cactgtttcg acaaaatcaa gaactggagg 720

aacctgatcg cggtgctggg cgagcacgac ctcagcgagc acgacgggga tgagcagagc 780

cggcgggtgg cgcaggtcat catccccagc acgtacgtcc cgggcaccac caaccacgac 840

atcgcgctgc tccgcctgca ccagcccgtg gtcctcactg accatgtggt gcccctctgc 900

ctgcccgaac ggacgttctc tgagaggacg ctggccttcg tgcgcttctc attggtcagc 960

ggctggggcc agctgctgga ccgtggcgcc acggccctgg agctcatggt gctcaacgtg 1020

ccccggctga tgacccagga ctgcctgcag cagtcacgga aggtgggaga ctccccaaat 1080

atcacggagt acatgttctg tgccggctac tcggatggca gcaaggactc ctgcaagggg 1140

gacagtggag gcccacatgc cacccactac cggggcacgt ggtacctgac gggcatcgtc 1200

agctggggcc agggctgcgc aaccgtgggc cactttgggg tgtacaccag ggtctcccag 1260

tacatcgagt ggctgcaaaa gctcatgcgc tcagagccac gcccaggagt cctcctgcga 1320

gccccatttc cctag 1335

<210> 23
<211> 1386
<212> DNA
<213> human

<400> 23
atgcagcgcg tgaacatgat catggcagaa tcaccaggcc tcatcaccat ctgcctttta 60

ggatatctac tcagtgctga atgtacagtt tttcttgatc atgaaaacgc caacaaaatt 120

ctgaatcggc caaagaggta taattcaggt aaattggaag agtttgttca agggaacctt 180

gagagagaat gtatggaaga aaagtgtagt tttgaagaag cacgagaagt ttttgaaaac 240

actgaaagaa caactgaatt ttggaagcag tatgttgatg gagatcagtg tgagtccaat 300

ccatgtttaa atggcggcag ttgcaaggat gacattaatt cctatgaatg ttggtgtccc 360

tttggatttg aaggaaagaa ctgtgaatta gatgtaacat gtaacattaa gaatggcaga 420

tgcgagcagt tttgtaaaaa tagtgctgat aacaaggtgg tttgctcctg tactgaggga 480

tatcgacttg cagaaaacca gaagtcctgt gaaccagcag tgccatttcc atgtggaaga 540

gtttctgttt cacaaacttc taagctcacc cgtgctgaga ctgtttttcc tgatgtggac 600

tatgtaaatt ctactgaagc tgaaaccatt ttggataaca tcactcaaag cacccaatca 660

tttaatgact tcactcgggt tgttggtgga gaagatgcca aaccaggtca attcccttgg 720

caggttgttt tgaatggtaa agttgatgca ttctgtggag gctctatcgt taatgaaaaa 780

tggattgtaa ctgctgccca ctgtgttgaa actggtgtta aaattacagt tgtcgcaggt 840

gaacataata ttgaggagac agaacataca gagcaaaagc gaaatgtgat tcgaattatt 900

cctcaccaca actacaatgc agctattaat aagtacaacc atgacattgc ccttctggaa 960

ctggacgaac ccttagtgct aaacagctac gttacaccta tttgcattgc tgacaaggaa 1020

tacacgaaca tcttcctcaa atttggatct ggctatgtaa gtggctgggg aagagtcttc 1080

cacaaaggga gatcagcttt agttcttcag taccttagag ttccacttgt tgaccgagcc 1140

acatgtcttc gatctacaaa gttcaccatc tataacaaca tgttctgtgc tggcttccat 1200

gaaggaggta gagattcatg tcaaggagat agtgggggac cccatgttac tgaagtggaa 1260

gggaccagtt tcttaactgg aattattagc tggggtgaag agtgtgcaat gaaaggcaaa 1320

tatggaatat ataccaaggt atcccggtat gtcaactgga ttaaggaaaa aacaaagctc 1380

acttaa 1386

<210> 24
<211> 1830
<212> DNA
<213> human

<400> 24
atgaagtggg taacctttat ttcccttctt tttctcttta gctcggctta ttccaggggt 60

gtgtttcgtc gagatgcaca caagagtgag gttgctcatc ggtttaaaga tttgggagaa 120

gaaaatttca aagccttggt gttgattgcc tttgctcagt atcttcagca gtgtccattt 180

gaagatcatg taaaattagt gaatgaagta actgaatttg caaaaacatg tgttgctgat 240

gagtcagctg aaaattgtga caaatcactt catacccttt ttggagacaa attatgcaca 300

gttgcaactc ttcgtgaaac ctatggtgaa atggctgact gctgtgcaaa acaagaacct 360

gagagaaatg aatgcttctt gcaacacaaa gatgacaacc caaacctccc ccgattggtg 420

agaccagagg ttgatgtgat gtgcactgct tttcatgaca atgaagagac atttttgaaa 480

aaatacttat atgaaattgc cagaagacat ccttactttt atgccccgga actccttttc 540

tttgctaaaa ggtataaagc tgcttttaca gaatgttgcc aagctgctga taaagctgcc 600

tgcctgttgc caaagctcga tgaacttcgg gatgaaggga aggcttcgtc tgccaaacag 660

agactcaagt gtgccagtct ccaaaaattt ggagaaagag ctttcaaagc atgggcagta 720

gctcgcctga gccagagatt tcccaaagct gagtttgcag aagtttccaa gttagtgaca 780

gatcttacca aagtccacac ggaatgctgc catggagatc tgcttgaatg tgctgatgac 840

agggcggacc ttgccaagta tatctgtgaa aatcaagatt cgatctccag taaactgaag 900

gaatgctgtg aaaaacctct gttggaaaaa tcccactgca ttgccgaagt ggaaaatgat 960

gagatgcctg ctgacttgcc ttcattagct gctgattttg ttgaaagtaa ggatgtttgc   1020

aaaaactatg ctgaggcaaa ggatgtcttc ctgggcatgt ttttgtatga atatgcaaga   1080

aggcatcctg attactctgt cgtgctgctg ctgagacttg ccaagacata tgaaaccact   1140

ctagagaagt gctgtgccgc tgcagatcct catgaatgct atgccaaagt gttcgatgaa   1200

tttaaacctc ttgtggaaga gcctcagaat ttaatcaaac aaaattgtga gctttttgag   1260

cagcttggag agtacaaatt ccagaatgcg ctattagttc gttacaccaa gaaagtaccc   1320

caagtgtcaa ctccaactct tgtagaggtc tcaagaaacc taggaaaagt gggcagcaaa   1380

tgttgtaaac atcctgaagc aaaaagaatg ccctgtgcag aagactatct atccgtggtc   1440

ctgaaccagt tatgtgtgtt gcatgagaaa acgccagtaa gtgacagagt caccaaatgc   1500

tgcacagaat ccttggtgaa caggcgacca tgcttttcag ctctggaagt cgatgaaaca   1560

tacgttccca aagagtttaa tgctgaaaca ttcaccttcc atgcagatat atgcacactt   1620

tctgagaagg agagacaaat caagaaacaa actgcacttg ttgagctcgt gaaacacaag   1680

cccaaggcaa caaaagagca actgaaagct gttatggatg atttcgcagc ttttgtagag   1740

aagtgctgca aggctgacga taaggagacc tgctttgccg aggagggtaa aaaacttgtt   1800

gctgcaagtc aagctgcctt aggcttataa                                    1830

<210>  25
<211>  27
<212>  DNA
<213>  artificial

<220>
<223>  Mutagenesis primer

<400>  25
ccccatttcc cgatgcacac aagagtg                                         27

<210>  26
<211>  27
<212>  DNA
<213>  artificial

<220>
<223>  Mutagenesis primer

<400>  26
cactcttgtg tgcatcggga aatgggg                                         27

<210>  27
<211>  21
<212>  DNA
<213>  artificial

<220>
<223>  PCR primer

<400>  27
ccactttcac aatctgctag c                                               21

<210>  28
<211>  23

<212>  DNA
<213>  artificial

<220>
<223>  PCR primer

<400>  28
caattccaat gaattaacct tgg                                              23


<210>  29
<211>  21
<212>  DNA
<213>  artificial

<220>
<223>  PCR primer

<400>  29
atgcagcgcg tgaacatgat c                                                21


<210>  30
<211>  25
<212>  DNA
<213>  artificial

<220>
<223>  PCR primer

<400>  30
tcattaagtg agctttgttt tttcc                                            25


<210>  31
<211>  21
<212>  DNA
<213>  artificial

<220>
<223>  PCR primer

<400>  31
gattcgaatt cgcccttatg c                                                21


<210>  32
<211>  32
<212>  DNA
<213>  artificial

<220>
<223>  PCR primer

<400>  32
cgctcgaggt gagctttgtt ttttccttaa tc                                    32


<210>  33
<211>  46
<212>  DNA
<213>  artificial

<220>
<223>  PCR primer

<400>  33
ggatccagat cccccagagc ctccagagcc tcccgaccct ccagag                     46

<210>  34
<211>  18
<212>  DNA
<213>  artificial

<220>
<223>  PCR primer

<400>  34
caaggagacg ggcgctcc                                                    18


<210>  35
<211>  19
<212>  DNA
<213>  artificial

<220>
<223>  PCR primer

<400>  35
gcccaaggag gggattggc                                                   19


<210>  36
<211>  30
<212>  DNA
<213>  artificial

<220>
<223>  PCR primer

<400>  36
gtggaattca tggagctgag gccctggttg                                       30


<210>  37
<211>  38
<212>  DNA
<213>  artificial

<220>
<223>  PCR primer

<400>  37
cacgcggccg ctcactacag ccgttgcccc gcctccac                              38


<210>  38
<211>  704
<212>  PRT
<213>  human

<400>  38

Met Glu Leu Arg Pro Trp Leu Leu Trp Val Val Ala Ala Thr Gly Thr
1               5                   10                  15


Leu Val Leu Leu Ala Ala Asp Ala Gln Gly Gln Lys Val Phe Thr Asn
            20                  25                  30


Thr Trp Ala Val Arg Ile Pro Gly Gly Pro Ala Val Ala Asn Ser Val
        35                  40                  45


Ala Arg Lys His Gly Phe Leu Asn Leu Gly Gln Ile Phe Gly Asp Tyr

50                  55                  60

Tyr His Phe Trp His Arg Gly Val Thr Lys Arg Ser Leu Ser Pro His
65                  70                  75                  80

Arg Pro Arg His Ser Arg Leu Gln Arg Glu Pro Gln Val Gln Trp Leu
                85                  90                  95

Glu Gln Gln Val Ala Lys Arg Arg Thr Lys Arg Asp Val Tyr Gln Glu
            100                 105                 110

Pro Thr Asp Pro Lys Phe Pro Gln Gln Trp Tyr Leu Ser Gly Val Thr
        115                 120                 125

Gln Arg Asp Leu Asn Val Lys Ala Ala Trp Ala Gln Gly Tyr Thr Gly
    130                 135                 140

His Gly Ile Val Val Ser Ile Leu Asp Asp Gly Ile Glu Lys Asn His
145                 150                 155                 160

Pro Asp Leu Ala Gly Asn Tyr Asp Pro Gly Ala Ser Phe Asp Val Asn
                165                 170                 175

Asp Gln Asp Pro Asp Pro Gln Pro Arg Tyr Thr Gln Met Asn Asp Asn
            180                 185                 190

Arg His Gly Thr Arg Cys Ala Gly Glu Val Ala Ala Val Ala Asn Asn
        195                 200                 205

Gly Val Cys Gly Val Gly Val Ala Tyr Asn Ala Arg Ile Gly Gly Val
    210                 215                 220

Arg Met Leu Asp Gly Glu Val Thr Asp Ala Val Glu Ala Arg Ser Leu
225                 230                 235                 240

Gly Leu Asn Pro Asn His Ile His Ile Tyr Ser Ala Ser Trp Gly Pro
                245                 250                 255

Glu Asp Asp Gly Lys Thr Val Asp Gly Pro Ala Arg Leu Ala Glu Glu
            260                 265                 270

Ala Phe Phe Arg Gly Val Ser Gln Gly Arg Gly Gly Leu Gly Ser Ile
        275                 280                 285

Phe Val Trp Ala Ser Gly Asn Gly Gly Arg Glu His Asp Ser Cys Asn
    290                 295                 300

Cys Asp Gly Tyr Thr Asn Ser Ile Tyr Thr Leu Ser Ile Ser Ser Ala
305                 310                 315                 320

Thr Gln Phe Gly Asn Val Pro Trp Tyr Ser Glu Ala Cys Ser Ser Thr
                325                 330                 335

Leu Ala Thr Thr Tyr Ser Ser Gly Asn Gln Asn Glu Lys Gln Ile Val
            340                 345                 350

Thr Thr Asp Leu Arg Gln Lys Cys Thr Glu Ser His Thr Gly Thr Ser
        355                 360                 365

Ala Ser Ala Pro Leu Ala Ala Gly Ile Ile Ala Leu Thr Leu Glu Ala
    370                 375                 380

Asn Lys Asn Leu Thr Trp Arg Asp Met Gln His Leu Val Val Gln Thr
385                 390                 395                 400

Ser Lys Pro Ala His Leu Asn Ala Asn Asp Trp Ala Thr Asn Gly Val
                405                 410                 415

Gly Arg Lys Val Ser His Ser Tyr Gly Tyr Gly Leu Leu Asp Ala Gly
            420                 425                 430

Ala Met Val Ala Leu Ala Gln Asn Trp Thr Thr Val Ala Pro Gln Arg
        435                 440                 445

Lys Cys Ile Ile Asp Ile Leu Thr Glu Pro Lys Asp Ile Gly Lys Arg
    450                 455                 460

Leu Glu Val Arg Lys Thr Val Thr Ala Cys Leu Gly Glu Pro Asn His
465                 470                 475                 480

Ile Thr Arg Leu Glu His Ala Gln Ala Arg Leu Thr Leu Ser Tyr Asn
                485                 490                 495

Arg Arg Gly Asp Leu Ala Ile His Leu Val Ser Pro Met Gly Thr Arg
            500                 505                 510

Ser Thr Leu Leu Ala Ala Arg Pro His Asp Tyr Ser Ala Asp Gly Phe
        515                 520                 525

Asn Asp Trp Ala Phe Thr Thr Thr His Ser Trp Asp Glu Asp Pro Ser
    530                 535                 540

Gly Glu Trp Val Leu Glu Ile Glu Asn Thr Ser Glu Ala Asn Asn Tyr
545                 550                 555                 560

Gly Thr Leu Thr Lys Phe Thr Leu Val Leu Tyr Gly Thr Ala Pro Glu
                565                 570                 575

Gly Leu Pro Val Pro Pro Glu Ser Ser Gly Cys Lys Thr Leu Thr Ser
            580                 585                 590

Ser Gln Ala Cys Val Val Cys Glu Glu Gly Phe Ser Leu His Gln Lys
        595                 600                 605

Ser Cys Val Gln His Cys Pro Pro Gly Phe Ala Pro Gln Val Leu Asp
    610                 615                 620

Thr His Tyr Ser Thr Glu Asn Asp Val Glu Thr Ile Arg Ala Ser Val
625                 630                 635                 640

Cys Ala Pro Cys His Ala Ser Cys Ala Thr Cys Gln Gly Pro Ala Leu
                645                 650                 655

Thr Asp Cys Leu Ser Cys Pro Ser His Ala Ser Leu Asp Pro Val Glu
            660                 665                 670

Gln Thr Cys Ser Arg Gln Ser Gln Ser Ser Arg Glu Ser Pro Pro Gln
        675                 680                 685

Gln Gln Pro Pro Arg Leu Pro Pro Glu Val Glu Ala Gly Gln Arg Leu
    690                 695                 700

## Description

### Field of the invention:

The present invention relates to the field of modified therapeutic polypeptides with increased in vivo recovery compared to their non-modified parent polypeptide. I.e., the invention relates to fusions of therapeutic polypeptides with recovery enhancing polypeptides connected directly or optionally connected by a linker peptide.

The gist of the invention is demonstrated in particular by vitamin K-dependent polypeptides like e.g. human Factor VII, human Factor VIIa, human Factor IX, and human protein C as the therapeutic polypeptide and albumin as the recovery enhancing polypeptide. Therefore, in particular, the invention also relates to cDNA sequences coding for any of the vitamin K-dependent polypeptides and derivatives genetically fused to a cDNA coding for human serum albumin which may be linked by oligonucleotides which code for intervening peptidic linkers, such encoded derivatives exhibiting improved in vivo recovery, recombinant expression vectors containing such cDNA sequences, host cells transformed with such recombinant expression vectors, recombinant polypeptides and derivatives which do have biological activities comparable to the unmodified wild type polypeptide but having improved in vivo recovery and processes for the manufacture of such recombinant polypeptides and their derivatives. The invention also covers a transfer vector for use in human gene therapy, which comprises such modified DNA sequences useful to increase product levels in vivo.

### Background of the invention:

### Therapeutic polypeptides

Therapeutic polypeptides in the sense of this invention are proteins or polypeptides that upon application to a human or animal can produce a prophylactic or therapeutic effect. These therapeutic polypeptides are applied to a human or an animal via oral, topical, parenteral or other routes. Specific classes of therapeutic polypeptides covered, i.e. by the examples in this invention, are vitamin K-dependent polypeptides that to some extent are commercially available in their plasma derived or recombinant version.

### Recovery enhancing polypeptides

Recovery enhancing polypeptides in the sense of this invention are any polypeptides or proteins, which upon fusion to a therapeutic polypeptide increase the in vivo recovery of the fusion in comparison to the non-modified therapeutic polypeptide. Specific examples of such recovery enhancing polypeptides are albumin, variants or fragments thereof, and immunoglobulins, variants or fragments thereof.

### In vivo recovery

In vivo recovery is defined as the percentage of therapeutic polypeptide, which is detectable in the circulation after a short period of time post application (5-10 minutes) in relation to the total amount of therapeutic polypeptide administered. As a basis for calculation of the expected therapeutic polypeptide concentration in the circulation a plasma volume of 40 mL per kg is assumed in general.

### Fusion proteins or fusion polypeptides

Fusion proteins or fusion polypeptides in the sense of this invention are proteins which can be expressed from genetic constructs comprising a nucleic acid coding for a therapeutic polypeptide or variants thereof and a nucleic acid coding for a recovery enhancing polypeptide in which construct both nucleic acids are linked in frame in a way that expression in a host cell in which said genetic construct is introduced, generates a protein in which the therapeutic polypeptide is linked by peptide linkage to the recovery enhancing polypeptide. Optionally the therapeutic polypeptide and the recovery enhancing polypeptide can also be connected by a short peptidic linker.

### Vitamin K-dependent polypeptides

Vitamin K-dependent polypeptides which are posttranslational modified by gamma-carboxylation and comprise e.g. the blood coagulation factors II (prothrombin), VII, IX, and X, the anticoagulant proteins C and S, and the thrombin-targeting protein Z, the bone protein osteocalcin, the calcification inhibiting matrix protein, the cell growth regulating growth arrest specific gene 6 protein (Gas6), and the four transmembrane Gla proteins (TMGPs) the function of which is at present unknown. Among those polypeptides some are used to treat certain types of hemophilia and bleeding disorders. Hemophilia A is an inherited bleeding disorder. It results from a chromosome X-linked deficiency of blood coagulation Factor VIII and the clinical manifestation is an increased bleeding tendency. The disease is treated by injection of FVIII concentrates from plasma or recombinant sources. Hemophilia B is caused by non-functional or missing Factor IX and is treated with Factor IX concentrates from plasma or a recombinant form of Factor IX. In both hemophilia A and in hemophilia B the most serious medical problem in treating the disease is the generation of alloantibodies against the replacement factors. Up to about 30% of all hemophilia A patients develop antibodies to Factor VIII. Antibodies to Factor IX are less frequent.

The current model of coagulation states that the physiological trigger of coagulation is the formation of a complex between tissue Factor (TF) and Factor VIIa (FVIIa) on the surface of TF expressing cells, which are normally located outside the vasculature. This leads to the activation of Factor IX and Factor X ultimately generating some thrombin. In a positive feedback loop thrombin -directly or indirectly- activates Factor VIII and Factor IX, the so-called "intrinsic" arm of the blood coagulation cascade, thus amplifying the generation of Factor Xa, which is necessary for the generation of the full thrombin burst to achieve complete hemostasis. It was shown that by administering supraphysiological concentrations of Factor VIIa hemostasis can be achieved bypassing the need for Factor VIIIa and Factor IXa. The cloning of the cDNA for Factor VII (US 4,784,950) made it possible to develop activated Factor VII as a pharmaceutical. Factor VIIa was successfully administered for the first time in 1988. Ever since the number of indications of Factor VIIa has grown steadily showing a potential to become a universal hemostatic agent to stop bleeding (Erhardtsen, 2002). However, the short half-life of Factor VIIa of approximately 2 hours and reduced in vivo recovery is limiting its application.

### Factor VII and Factor VIIa

FVII is a single-chain glycoprotein with a molecular weight of 50 kDa, which is secreted by liver cells into the blood stream as an inactive zymogen of 406 amino acids. It contains 10 γ-carboxy-glutamic acid residues localized in the N-terminal Gla-domain of the polypeptide. The Gla residues require vitamin K for their biosynthesis. Located C-terminal to the Gla domain are two epidermal growth factor domains followed by a trypsin-type serine protease domain. Further posttranslational modifications of FVII encompass hydroxylation (Asp 63), N-(Asn145 and Asn322) as well as O-type glycosylation (Ser52 and Ser60).

FVII is converted to its active form Factor VIIa by proteolysis of the single peptide bond at Arg152-IIe153 leading to the formation of two polypeptide chains, a N-terminal light chain (24 kDa) and a C-terminal heavy chain (28 kDa), which are held together by one disulfide bridge. In contrast to other vitamin K-dependent coagulation factors, no activation peptide that is cleaved off during activation of these other vitamin-K dependent coagulation factors has been described for FVII. Essential for attaining the active conformation of Factor VIIa is the formation of a salt bridge after activation cleavage between IIe153 and Asp343. Activation cleavage of Factor VII can be achieved in vitro by Factor Xa, Factor XIIa, Factor IXa, Factor VIIa, Factor Seven Activating Protease (FSAP) and thrombin. Mollerup et al. (Biotechnol. Bioeng. (1995) 48: 501-505) reported that some cleavage also occurs in the heavy chain at Arg290 and or Arg315.

Factor VII is present in plasma in a concentration of about 500 ng/ml. About 1% or 5 ng/ml of Factor VII are present as Factor VIIa. Plasma half-life of Factor VII was found to be about 4 hours and that of Factor VIIa about 2 hours. The half-life of Factor VIIa of 2 hours constitutes a severe drawback for the therapeutic use of Factor VIIa, as it leads to the need of multiple i.v. injections or continuous infusion to achieve hemostasis. This results in very high treatment cost and inconvenience for the patient. Both, improvement in plasma half-life and in vivo recovery, would bring benefit to the patient. Up to now no pharmaceutical preparation of a Factor VIIa with improved in vivo recovery is commercially available nor have any data been published showing FVII/FVIIa variants with improved in vivo recovery. As Factor VII/VIIa has the potential to be used as a universal hemostatic agent, a high medical need still exists to develop forms of Factor VIIa which have an improved in vivo recovery.

### Factor IX

Human FIX is a single-chain glycoprotein with a molecular weight of 57 kDa, which is secreted by liver cells into the blood stream as an inactive zymogen of 415 amino acids. It contains 12 γ-carboxy-glutamic acid residues localized in the N-terminal Gla-domain of the polypeptide. The Gla residues require vitamin K for their biosynthesis. Located C-terminal to the Gla domain are two epidermal growth factor domains and an activation peptide followed by a trypsin-type serine protease domain. Further posttranslational modifications of FIX encompass hydroxylation (Asp 64), N- (Asn157 and Asn167) as well as O-type glycosylation (Ser53, Ser61, Thr159, Thr169, and Thr172), sulfation (Tyr155), and phosphorylation (Ser158).

FIX is converted to its active form Factor IXa by proteolysis of the activation peptide at Arg145-Ala146 and Arg180-Val181 leading to the formation of two polypeptide chains, a N-terminal light chain (18 kDa) and a C-terminal heavy chain (28 kDa), which are held together by one disulfide bridge. Activation cleavage of Factor IX can be achieved in vitro e.g. by Factor XIa or Factor VIIa/TF.

Factor IX is present in human plasma in a concentration of 5-10 µg/ml. Plasma half-life of Factor IX in humans was found to be about 15-18 hours (White GC et al. 1997. Thromb Haemost. 78:261-265; Ewenstein BM et al. 2002. Transfusion 42:190-197).

As haemophilia B patients often receive biweekly prophylactic administrations of Factor IX to avoid spontaneous bleedings, it is desirable to reduce the intervals of application by increasing the vivo recovery of the Factor IX product applied. Both, improvement in plasma half-life and in vivo recovery, would bring significant benefit to the patient. Up to now no pharmaceutical preparation of a Factor IX with improved plasma half-life or in vivo recovery is commercially available nor have any data been published showing Factor IX variants with prolonged in vivo half-life and improved in vivo recovery. Therefore, a high medical need still exists to develop forms of Factor IX which have a longer functional half-life in vivo and/or an improved in vivo recovery.

Recombinant therapeutic polypeptide drugs are usually expensive and not all countries can afford costly therapies based on such drugs. Increasing the in vivo recovery of such drugs will also make state of the art treatment cheaper and subsequently more patients will benefit from it.

Ballance et al. (WO 01/79271) describe fusion polypeptides of a multitude of different therapeutic proteins which, when fused to human serum albumin, are predicted to have increased functional half-life in vivo and extended shelf-life. Long lists of potential fusion partners are described without showing by experimental data for almost all of these proteins that the respective albumin fusion polypeptides actually retain biological activity and have improved properties. Among said list of therapeutic polypeptides also Factor IX and FVII/FVIIa are mentioned as examples of the invention. Ballance et al. is silent about in the vivo recovery of such fusion proteins.

### In vivo recovery of vitamin K-dependent polypeptides

In vivo recovery of recombinant FIX (BeneFIX, Genetics Institute) of 0.84 - 0.86 IU/dL per IU/kg has been reported to be significantly lower in haemophilia B patients than that of plasma derived FIX like Mononine of 1.17 - 1.71 IU/dL per IU/kg (White G. et al., Semin Hematol 35 (Suppl. 2): 33-38 (1998); Ewenstein B.M. et al., Transfusion 42(2): 190-197 (2002)). As a consequence, at least 20% higher amounts of recombinant FIX have to be applied in comparison to plasma derived FIX to achieve comparably efficient treatment of haemophilia B.

Sheffield (Sheffield WP et al. (2004) Br. J. Haematol. 126:565-573) expressed a human Factor IX albumin fusion polypeptide and showed in pharmacokinetic experiments that in FIX knockout mice, the in vivo recovery of the human FIX-albumin fusion protein was significantly lower (less than half) than the unfused human FIX molecule.

In vivo recovery of recombinant FVIIa (NovoSeven, Novo Nordisk) has been reported to be about 19 to 22% in FVII deficient patients (Berrettini M et al. 2001. Haematologica 86:640-645) and about 46-48% in hemophilia patients (Lindley CM et al, 1994. Clin. Pharmacol. Ther. 55:638-648). Likewise the in vivo recovery of rFVIIa was described at about 34% in hemophilia A dogs and about 44% in hemophilia B dogs, respectively (Brinkhous KM et al., 1989. Proc. Natl. Acad. Sci. 86:1382-1386).

### GIST OF THE INVENTION:

As therapeutic polypeptides in general are rather expensive due to their costly manufacturing processes, an increase in the in vivo recovery would help to provide such products at a cheaper price and to treat more people than currently possible. In addition, a reduced frequency of applications would improve the convenience for the patients.

Therefore, the technical problem underlying the present invention was to develop therapeutic polypeptides, in particularly vitamin K dependent polypeptides, which show increased in vivo recovery and, therefore, facilitate the reduction of the dose or the frequency the product is applied.

### SUMMARY OF THE INVENTION:

Surprisingly it was found that vitamin K-dependent polypeptides when expressed as fusion proteins with albumin exhibit improved in vivo recoveries. By way of non limiting example we found that in contrast to the results with a Factor IX albumin fusion protein published by Sheffield et al. (Sheffield WP et al. (2004) Br. J. Haematol. 126:565-573) human FIX albumin fusion proteins exhibit improved in vivo recovery compared to the unfused Factor IX. It was further found that fusions of Factor VII/VIIa to human serum albumin led to Factor VII/FVIIa fusion proteins, which retained Factor VII/FVIIa biological activity and displayed an increased in vivo recovery.

One aspect of the invention are therefore therapeutic polypeptides fused to the Nor C-terminus of albumin or any other recovery enhancing polypeptide, in which the fusion proteins display at least 110%, preferably more than 125%, even more preferably more than 140% of the in vivo recovery of the respective recombinantly produced non-fused therapeutic polypeptide or peptide.

Another aspect of the invention are vitamin K-dependent polypeptides fused to the N- or C-terminus of albumin or any other recovery enhancing polypeptide. The fusion proteins display a significant increase of the in vivo recovery of the respective recombinantly produced, wild-type vitamin K dependent polypeptides.

A further aspect of the invention are fusion proteins in which Factor VII/VIIa polypeptides are fused to the N-terminus of albumin which display a significant increase of the in vivo recovery as compared to unfused, recombinantly produced Factor VII/VIIa.

Another aspect of the invention are fusion proteins in which Factor IX polypeptides are fused to the N-terminus of albumin which display a significant increase of the in vivo recovery as compared to unfused Factor IX.

One aspect of the invention are therefore vitamin K dependent polypeptides fused to the N- or C-terminus of albumin increasing the in vivo recovery compared to the corresponding recombinant non-fused polypeptide by at least 10%, preferably more than 25%, even more preferably more than 40%.

The invention encompasses therapeutic polypeptides, in particular vitamin K dependent polypeptides linked to the N- or C-terminus of a recovery enhancing polypeptide like albumin, compositions, pharmaceutical compositions, formulations and kits. The invention also encompasses the use of said recovery enhancing polypeptide linked therapeutic polypeptides in certain medical indications in which the unfused therapeutic polypeptides also would be applicable. The invention also encompasses nucleic acid molecules encoding the recovery enhancing polypeptides linked therapeutic polypeptides of the invention, as well as vectors containing these nucleic acids, host cells transformed with these nucleic acids and vectors, and methods of making the recovery enhancing polypeptides linked therapeutic polypeptides of the invention using these nucleic acids, vectors, and/or host cells.

The invention also provides a composition comprising a vitamin K dependent polypeptide, or a fragment or variant thereof, optionally a peptidic linker, and albumin, or a fragment or variant thereof, and a pharmaceutically acceptable carrier. Another objective of the invention is to provide a method of treating patients with bleeding disorders. The method comprises the step of administering an effective amount of the fusion polypeptide including the vitamin K dependent polypeptide.

Another aspect of the invention is to provide a nucleic acid molecule comprising a polynucleotide sequence encoding albumin fusion polypeptide comprising a vitamin K dependent polypeptide, or a fragment or variant thereof, optionally a peptidic linker, and albumin, or a fragment or variant thereof, as well as a vector that comprises such a nucleic acid molecule.

The invention also provides a method for manufacturing an albumin fusion polypeptide comprising a vitamin K dependent polypeptide, or a fragment or variant thereof, a peptidic linker, and albumin, or a fragment or variant thereof, wherein the method comprises:
(a) providing a nucleic acid comprising a nucleotide sequence encoding the vitamin K dependent polypeptide linked to the albumin polypeptide expressible in a mammalian cell;
(b) expressing the nucleic acid in the organism to form a vitamin K dependent polypeptide linked to the albumin polypeptide; and
(c) purifying the vitamin K dependent polypeptide linked to albumin polypeptide.

An albumin fusion polypeptide of the present invention preferably comprises at least a fragment or variant of a vitamin K dependent polypeptide and at least a fragment or variant of human serum albumin, which are associated with one another, such as by genetic fusion (i.e. the albumin fusion polypeptide is generated by translation of a nucleic acid in which a polynucleotide encoding all or a portion of a vitamin K dependent polypeptide is joined in-frame to the 5'end of a polynucleotide encoding all or a portion of albumin optionally linked by a polynucleotide which encodes a linker sequence, introducing a linker peptide between the vitamin K dependent polypeptide moiety and the albumin moiety).

In one embodiment, the invention provides a vitamin K dependent polypeptide albumin fusion polypeptide comprising, or alternatively consisting of biologically active or activatable and/or therapeutically active or activatable vitamin K dependent polypeptide fused to the N-terminus of a serum albumin polypeptide.

In other embodiments, the invention provides an albumin fusion polypeptide comprising, or alternatively consisting of, a biologically active or activatable and/or therapeutically active or activatable fragment of a vitamin K dependent polypeptide and a peptidic linker fused to the N-terminus of a serum albumin.

In other embodiments, the invention provides a vitamin K dependent polypeptide albumin fusion polypeptide comprising, or alternatively consisting of, a biologically active or activatable and/or therapeutically active or activatable variant of a vitamin K dependent polypeptide fused to the N-terminus of a serum albumin polypeptide and optionally a peptidic linker.

In further embodiments, the invention provides a vitamin K dependent polypeptide albumin fusion polypeptide comprising, or alternatively consisting of, a biologically active or activatable and/or therapeutically active or activatable fragment or variant of a vitamin K dependent polypeptide fused to the N-terminus of a fragment or variant of serum albumin and optionally a peptidic linker.

In some embodiments, the invention provides an albumin fusion polypeptide comprising, or alternatively consisting of, the mature portion of a vitamin K dependent polypeptide fused to the N-terminus of the mature portion of serum albumin and optionally a peptidic linker.
The fusion proteins of the present invention may be used therapeutically in all those indications the non-fused polypeptides or proteins can be applied.

### DETAILED DESCRIPTION OF THE INVENTION:

It is an objective of the present invention to provide a method to increase the in vivo recovery of therapeutic polypeptides as compared to unfused therapeutic polypeptides, in particular vitamin K dependent polypeptides or fragments or variants thereof by fusion to the N- or C-terminus of a recovery enhancing polypeptide like human albumin or fragments or variants thereof. As nonlimiting examples of the invention, fusions of therapeutic polypeptides, in particular vitamin K dependent polypeptides, to the N-terminus of serum albumin are provided optionally with an intervening peptidic linker between the vitamin K dependent polypeptide and albumin.

The terms, human serum albumin (HSA) and human albumin (HA) are used interchangeably herein. The terms "albumin" and "serum albumin" are broader, and encompass human serum albumin (and fragments and variants thereof) as well as albumin from other species (and fragments and variants thereof).

As used herein, "albumin" refers collectively to albumin polypeptide or amino acid sequence, or an albumin fragment or variant, having one or more functional activities (e.g., biological activities) of albumin. In particular, "albumin" refers to human albumin or fragments thereof especially the mature form of human albumin as shown in SEQ ID No: 20 herein or albumin from other vertebrates or fragments thereof, or analogs or variants of these molecules or fragments thereof.

The albumin portion of the albumin linked polypeptides may comprise the full length of the HA sequence as described above, or may include one or more fragments thereof that are capable of stabilizing or prolonging the therapeutic activity. Such fragments may be of 10 or more amino acids in length or may include about 15, 20, 25, 30, 50, or more contiguous amino acids from the HA sequence or may include part or all of specific domains of HA.

The albumin portion of the albumin-linked polypeptides of the invention may be a variant of normal HA. The vitamin K dependent polypeptide portion of the albumin-linked polypeptides of the invention may also be variants of the vitamin K dependent polypeptides as described herein. The term "variants" includes insertions, deletions and substitutions, either conservative or non-conservative, where such changes do not substantially alter the active site, or active domain which confers the therapeutic activities of the vitamin K dependent polypeptides.

In particular, the albumin-linked polypeptides of the invention may include naturally occurring polymorphic variants of human albumin and fragments of human albumin. The albumin may be derived from any vertebrate, especially any mammal, for example human, cow, sheep, or pig. Non-mammalian albumins include, but are not limited to, hen and salmon. The albumin portion of the albumin-linked polypeptide may be from a different animal than the vitamin K dependent polypeptide portion.

Generally speaking, an albumin fragment or variant will be at least 20, preferably at least 40, most preferably more than 70 amino acids long. The albumin variant may preferentially consist of or alternatively comprise at least one whole domain of albumin or fragments of said domains, for example domains 1 (amino acids 1-194 of SEQ ID NO:20), 2 (amino acids 195-387 of SEQ ID NO: 20), 3 (amino acids 388-585 of SEQ ID NO: 20), 1 + 2 (1-387 of SEQ ID NO: 20), 2 + 3 (195-585 of SEQ ID NO: 20) or 1 + 3 (amino acids 1-194 of SEQ ID NO: 20 + amino acids 388-585 of SEQ ID NO: 20). Each domain is itself made up of two homologous subdomains namely 1-105, 120-194, 195-291, 316-387, 388-491 and 512-585, with flexible inter-subdomain linker regions comprising residues Lys106 to Glu119, Glu292 to Val315 and Glu492 to Ala511.

The albumin portion of an albumin fusion polypeptide of the invention may comprise at least one subdomain or domain of HA or conservative modifications thereof.

The invention relates to a modified vitamin K dependent polypeptide, comprising linking the vitamin K dependent polypeptide or fragment or variant thereof to the Nor C-terminus of an albumin polypeptide or fragment or variant thereof optionally such that an intervening peptidic linker is introduced between the modified vitamin K dependent polypeptide and albumin such that the modified vitamin K dependent polypeptide has an increased in vivo recovery compared to the vitamin K dependent polypeptide which has not been linked to albumin.

"Vitamin K dependent polypeptide" as used in this application include, but are not limited to, a therapeutic polypeptide consisting of Factor VII, Factor VIIa, Factor IX, Factor IXa, Factor X, Factor Xa, Factor II (Prothrombin), Protein C, activated Protein C, Protein S, activated Protein S, GAS6, activated GAS6, Protein Z, activated Protein Z, and the like. Furthermore, useful vitamin K dependent polypeptides can be wild-type or can contain mutations. Degree and location of glycosylation or other post-translation modifications may vary depending on the chosen host cells and the nature of the host cellular environment. When referring to specific amino acid sequences, posttranslational modifications of such sequences are encompassed in this application.

"Vitamin K dependent polypeptides" within the above definition includes polypeptides that have the natural amino acid sequence. It also includes polypeptides with a slightly modified amino acid sequence, for instance, a modified N-terminal or C-terminal end including terminal amino acid deletions or additions as long as those polypeptides substantially retain the activity of the respective vitamin K dependent polypeptide. "Vitamin K dependent polypeptide" within the above definition also includes natural allelic variations that may exist and occur from one individual to another. "Vitamin K dependent polypeptide" within the above definition further includes variants of vitamin K dependent polypeptides. Such variants differ in one or more amino acid residues from the wild type sequence. Examples of such differences may include truncation of the N- and/or C-terminus by one or more amino acid residues (e.g. 1 to 10 amino acid residues), or addition of one or more extra residues at the N- and/or C-terminus, as well as conservative amino acid substitutions, i.e. substitutions performed within groups of amino acids with similar characteristics, e.g. (1) small amino acids, (2) acidic amino acids, (3) polar amino acids, (4) basic amino acids, (5) hydrophobic amino acids, and (6) aromatic amino acids. Examples of such conservative substitutions are shown in table 1.

**Table 1**

| | | | | |
|---|---|---|---|---|
| (1) | Alanine | Glycine | | |
| (2) | Aspartic acid | Glutamic acid | | |
| (3a) | Asparagine | Glutamine | | |
| (3b) | Serine | Threonine | | |
| (4) | Arginine | Histidine | Lysine | |
| (5) | Isoleucine | Leucine | Methionine | Valine |
| (6) | Phenylalanine | Tyrosine | Tryptophane | |

The vitamin K dependent polypeptide albumin fusions of the invention have at least 10%, preferably at least 25% and more preferably at least 40% increased in vivo recovery compared to unfused vitamin K dependent polypeptides.

The in vivo recovery of the Factor VII albumin linked polypeptides of the invention is usually at least about 10%, preferably at least about 25%, more preferably at least about 40% higher than the in vivo recovery of the wild type form of human Factor VII.

The in vivo recovery of the Factor VIIa albumin linked polypeptides of the invention is usually at least about 10%, preferably at least about 25%, more preferably at least about 40% higher than the in vivo recovery of the wild type form of human Factor VIIa.

The in vivo recovery of the Factor IX albumin linked polypeptides of the invention is usually at least about 10%, preferably at least about 25%, more preferably at least about 40% higher than the in vivo recovery of the wild type form of human Factor IX.

According to the invention the vitamin K dependent polypeptide moiety is coupled to the albumin moiety by a peptidic linker. The linker should be flexible and nonimmunogenic. Exemplary linkers include (GGGGS)ₙ or (GGGS)ₙ or (GGS)ₙ, wherein n is an integer greater than or equal to 1 and wherein G represents glycine and S represents serine.

In another embodiment of the invention the peptidic linker between the vitamin K dependent polypeptide moiety and the albumin moiety contains consensus sites for the addition of posttranslational modifications. Preferably such modifications consist of glycosylation sites. More preferably, such modifications consist of at least one N-glycosylation site of the structure Asn - X - Ser/Thr, wherein X denotes any amino acid except proline. Even more preferably such N-glycosylation sites are inserted close to the amino and/or carboxy terminus of the peptidic linker such that they are capable to shield potential neoepitopes which might develop at the sequences where the vitamin K dependent polypeptide moiety is transitioning into the peptidic linker and where the peptidic linker is transitioning into the albumin moiety sequence, respectively.

The invention further relates to a polynucleotide encoding a vitamin K dependent polypeptide albumin fusion as described in this application. The term "polynucleotide(s)" generally refers to any polyribonucleotide or polydeoxyribonucleotide that may be unmodified RNA or DNA or modified RNA or DNA. The polynucleotide may be single- or double-stranded DNA, single or double-stranded RNA. As used herein, the term "polynucleotide(s)" also includes DNAs or RNAs that comprise one or more modified bases and/or unusual bases, such as inosine. It will be appreciated that a variety of modifications may be made to DNA and RNA that serve many useful purposes known to those of skill in the art. The term "polynucleotide(s)" as it is employed herein embraces such chemically, enzymatically or metabolically modified forms of polynucleotides, as well as the chemical forms of DNA and RNA characteristic of viruses and cells, including, for example, simple and complex cells.

The skilled person will understand that, due to the degeneracy of the genetic code, a given polypeptide can be encoded by different polynucleotides. These "variants" are encompassed by this invention.

Preferably, the polynucleotide of the invention is an isolated polynucleotide. The term "isolated" polynucleotide refers to a polynucleotide that is substantially free from other nucleic acid sequences, such as and not limited to other chromosomal and extrachromosomal DNA and RNA. Isolated polynucleotides may be purified from a host cell. Conventional nucleic acid purification methods known to skilled artisans may be used to obtain isolated polynucleotides. The term also includes recombinant polynucleotides and chemically synthesized polynucleotides.

Yet another aspect of the invention is a plasmid or vector comprising a polynucleotide according to the invention. Preferably, the plasmid or vector is an expression vector. In a particular embodiment, the vector is a transfer vector for use in human gene therapy.

Still another aspect of the invention is a host cell comprising a polynucleotide of the invention or a plasmid or vector of the invention.

The host cells of the invention may be employed in a method of producing a vitamin K dependent polypeptide albumin fusion, which is part of this invention. The method comprises:
- culturing host cells of the invention under conditions such that the vitamin K dependent polypeptide albumin fusion is expressed; and
- optionally recovering the vitamin K dependent polypeptide albumin fusion from the culture medium.

### EXPRESSION OF THE PROPOSED POLYPEPTIDES:

The production of recombinant proteins at high levels in suitable host cells requires the assembly of the above-mentioned modified cDNAs into efficient transcriptional units together with suitable regulatory elements in a recombinant expression vector, that can be propagated in various expression systems according to methods known to those skilled in the art. Efficient transcriptional regulatory elements could be derived from viruses having animal cells as their natural hosts or from the chromosomal DNA of animal cells. Preferably, promoter-enhancer combinations derived from the Simian Virus 40, adenovirus, BK polyoma virus, human cytomegalovirus, or the long terminal repeat of Rous sarcoma virus, or promoter-enhancer combinations including strongly constitutively transcribed genes in animal cells like beta-actin or GRP78 can be used. In order to achieve stable high levels of mRNA transcribed from the cDNAs, the transcriptional unit should contain in its 3'-proximal part a DNA region encoding a transcriptional termination-polyadenylation sequence. Preferably, this sequence is derived from the Simian Virus 40 early transcriptional region, the rabbit beta-globin gene, or the human tissue plasminogen activator gene.

The cDNAs are then integrated into the genome of a suitable host cell line for expression of the therapeutic polypeptide albumin fusion polypeptides. Preferably this cell line should be an animal cell-line of vertebrate origin in order to ensure correct folding, gamma-carboxylation of glutamic acid residues within the Gla-domain, disulfide bond formation, asparagine-linked glycosylation, O-linked glycosylation, and other post-translational modifications as well as secretion into the cultivation medium. Examples of other post-translational modifications are tyrosine O-sulfation, hydroxylation, phosphorylation, proteolytic processing of the nascent polypeptide chain and cleavage of the propeptide region. Examples of cell lines that can be use are monkey COS-cells, mouse L-cells, mouse C127-cells, hamster BHK-21 cells, human embryonic kidney 293 cells, and hamster CHO-cells.

The recombinant expression vector encoding the corresponding cDNAs can be introduced into an animal cell line in several different ways. For instance, recombinant expression vectors can be created from vectors based on different animal viruses. Examples of these are vectors based on baculovirus, vaccinia virus, adenovirus, and preferably bovine papilloma virus.

The transcription units encoding the corresponding DNAs can also be introduced into animal cells together with another recombinant gene which may function as a dominant selectable marker in these cells in order to facilitate the isolation of specific cell clones which have integrated the recombinant DNA into their genome. Examples of this type of dominant selectable marker genes are Tn5 amino glycoside phosphotransferase, conferring resistance to geneticin (G418), hygromycin phosphotransferase, conferring resistance to hygromycin, and puromycin acetyl transferase, conferring resistance to puromycin. The recombinant expression vector encoding such a selectable marker can reside either on the same vector as the one encoding the cDNA of the desired polypeptide, or it can be encoded on a separate vector which is simultaneously introduced and integrated to the genome of the host cell, frequently resulting in a tight physical linkage between the different transcription units.

Other types of selectable marker genes which can be used together with the cDNA of the desired protein are based on various transcription units encoding dihydrofolate reductase (dhfr). After introduction of this type of gene into cells lacking endogenous dhfr-activity, preferentially CHO-cells (DUKX-B11, DG-44) it will enable these to grow in media lacking nucleosides. An example of such a medium is Ham's F12 without hypoxanthine, thymidin, and glycine. These dhfrgenes can be introduced together with the coagulation Factor cDNA transcriptional units into CHO-cells of the above type, either linked on the same vector or on different vectors, thus creating dhfr-positive cell lines producing recombinant protein.

If the above cell lines are grown in the presence of the cytotoxic dhfr-inhibitor methotrexate, new cell lines resistant to methotrexate will emerge. These cell lines may produce recombinant protein at an increased rate due to the amplified number of linked dhfr and the desired protein's transcriptional units. When propagating these cell lines in increasing concentrations of methotrexate (1-10000 nM), new cell lines can be obtained which produce the desired protein at very high rate.

The above cell lines producing the desired protein can be grown on a large scale, either in suspension culture or on various solid supports. Examples of these supports are micro carriers based on dextran or collagen matrices, or solid supports in the form of hollow fibres or various ceramic materials. When grown in cell suspension culture or on micro carriers the culture of the above cell lines can be performed either as a batch culture or as a perfusion culture with continuous production of conditioned medium over extended periods of time. Thus, according to the present invention, the above cell lines are well suited for the development of an industrial process for the production of the desired recombinant proteins

The recombinant protein, which accumulates in the medium of secreting cells of the above types, can be concentrated and purified by a variety of biochemical and chromatographic methods, including methods utilizing differences in size, charge, hydrophobicity, solubility, specific affinity, etc. between the desired protein and other substances in the cell cultivation medium.

An example of such purification is the adsorption of the recombinant protein to a monoclonal antibody or a binding peptide, which is immobilised on a solid support. After desorption, the protein can be further purified by a variety of chromatographic techniques based on the above properties.

It is preferred to purify the therapeutic polypeptide e.g. the vitamin K dependent polypeptide albumin fusion of the present invention to greater than 80% purity, more preferably greater than 95% purity, and particularly preferred is a pharmaceutically pure state that is greater than 99.9% pure with respect to contaminating macromolecules, particularly other proteins and nucleic acids, and free of infectious and pyrogenic agents. Preferably, an isolated or purified therapeutic polypeptide e.g. a vitamin K dependent polypeptide albumin fusion of the invention is substantially free of other polypeptides.

The therapeutic polypeptide, respectively vitamin K dependent polypeptide albumin fusion described in this invention can be formulated into pharmaceutical preparations for therapeutic use. The purified proteins may be dissolved in conventional physiologically compatible aqueous buffer solutions to which there may be added, optionally, pharmaceutical excipients to provide pharmaceutical preparations.

Such pharmaceutical carriers and excipients as well as suitable pharmaceutical formulations are well known in the art (see for example "Pharmaceutical Formulation Development of Peptides and Proteins", Frokjaer et al., Taylor & Francis (2000) or "Handbook of Pharmaceutical Excipients", 3rd edition, Kibbe et al., Pharmaceutical Press (2000)). In particular, the pharmaceutical composition comprising the polypeptide variant of the invention may be formulated in lyophilized or stable soluble form. The therapeutic polypeptide may be lyophilized by a variety of procedures known in the art. Lyophilized formulations are reconstituted prior to use by the addition of one or more pharmaceutically acceptable diluents such as sterile water for injection or sterile physiological saline solution.

Formulations of the composition are delivered to the individual by any pharmaceutically suitable means of administration. Various delivery systems are known and can be used to administer the composition by any convenient route. Preferentially the compositions of the invention are administered systemically. For systemic use, the albumin linked fusion proteins of the invention are formulated for parenteral (e.g. intravenous, subcutaneous, intramuscular, intraperitoneal, intracerebral, intrapulmonar, intranasal or transdermal) or enteral (e.g., oral, vaginal or rectal) delivery according to conventional methods. The most preferential route of administration is intravenous administration. The formulations can be administered continuously by infusion or by bolus injection. Some formulations encompass slow release systems.

The therapeutic polypeptides of the invention, respectively albumin-linked vitamin K dependent polypeptides of the present invention are administered to patients in a therapeutically effective dose, meaning a dose that is sufficient to produce the desired effects, preventing or lessening the severity or spread of the condition or indication being treated without reaching a dose which produces intolerable adverse side effects. The exact dose depends on many factors as e.g. the indication, formulation, and mode of administration and has to be determined in preclinical and clinical trials for each respective indication.

The pharmaceutical composition of the invention may be administered alone or in conjunction with other therapeutic agents. These agents may be incorporated as part of the same pharmaceutical.

The various products of the invention are useful as medicaments. Accordingly, the invention relates to a pharmaceutical composition comprising an albumin linked vitamin K dependent polypeptide as described herein, a polynucleotide of the invention, or a plasmid or vector of the invention.

The modified DNA's of this invention may also be integrated into a transfer vector for use in the human gene therapy.

Another aspect of the invention is the use of a therapeutic polypeptide of the invention e.g. an albumin-linked vitamin K dependent polypeptide as described herein, of a polynucleotide of the invention, of a plasmid or vector of the invention, or of a host cell of the invention for the manufacture of a medicament for the treatment or prevention of bleeding disorders. Bleeding disorders include but are not limited to hemophilia A. In another embodiment of the invention, the treatment comprises human gene therapy.

The invention also concerns a method of treating an individual in one or more of the following indications: "Haemophilia A or B", "bleeding episodes in patients with inherited or acquired coagulation deficiencies", "vascular occlusion episodes like e.g. thrombosis in patients with inherited or acquired factor deficiencies", "sepsis", "bleeding episodes and surgery in patients with inherited or acquired hemophilia with inhibitors to coagulation Factors (FVIII or FIX)", "reversal of hemostasis deficits developed as consequence of drug treatments such as anti-platelet drugs or anticoagulation drugs", "improvement of secondary hemostasis", "hemostasis deficits developed during infections or during illnesses such as Vitamin K deficiency or severe liver disease", "liver resection", "hemostasis deficits developed as consequences of snake bites", "gastro intestinal bleeds". Also preferred indications are "trauma", "consequences of massive transfusion (dilutional coagulopathy)", "coagulation factor deficiencies other than FVIII and FIX", "VWD", "FI deficiency", "FV deficiency", "FVII deficiency", "FX deficiency", "FXIII deficiency", "HUS", "inherited or acquired platelet diseases and disorders like thrombocytopenia, ITP, TTP, HELLP syndrome, Bernard-Soulier syndrome, Glanzmann Thrombasthenia, HIT", "Chediak-Higahi Syndrom", "Hermansky-Pudlak-Syndrome", "Rendu-Osler Syndrome", "Henoch-Schonlein purpura", "Wound Healing", and "Sepsis". The method comprises administering to said individual an efficient amount of the vitamin K-dependent albumin linked polypeptide as described herein. In another embodiment, the method comprises administering to the individual an efficient amount of the polynucleotide of the invention or of a plasmid or vector of the invention. Alternatively, the method may comprise administering to the individual an efficient amount of the host cells of the invention described herein.

### DESCRIPTION OF TABLES AND DRAWINGS:

**Figure 1****:**
   Xhol restriction site introduced at the site of the natural FVII stop codon by replacing TAG by TCG. Mutated base is indicated in bold letter. The Notl site used for further construction is double underlined. The amino acid sequence of the Factor VII C-terminus is given in three letter code (boxed).
**Figure 2****:**
   Outline of the linker sequences inserted between the C-terminus of Factor VII and the N-terminus of albumin in the various pFVII constructs. The asparagines of the N-glycosylation sites are double underlined.
**Figure 3****:**
   Outline of the linker sequences inserted between the C-terminus of Factor IX and the N-terminus of albumin in the various pFIX constructs. The asparagines of the N-glycosylation sites are double underlined.

### EXAMPLES:

### Example 1: Generation of cDNAs encoding FVII and FVII - albumin fusion proteins

Factor VII coding sequence was amplified by PCR from a human liver cDNA library (ProQuest, Invitrogen) using primers We1303 and We1304 (SEQ ID NO 1 and 2). After a second round of PCR using primers We1286 and We1287 (SEQ ID NO 3 and 4) the resulting fragment was cloned into pCR4TOPO (Invitrogen). From there the FVII cDNA was transferred as an EcoRI Fragment into the EcoRI site of pIRESpuro3 (BD Biosciences) wherein an internal Xhol site had been deleted previously. The resulting plasmid was designated pFVII-659.

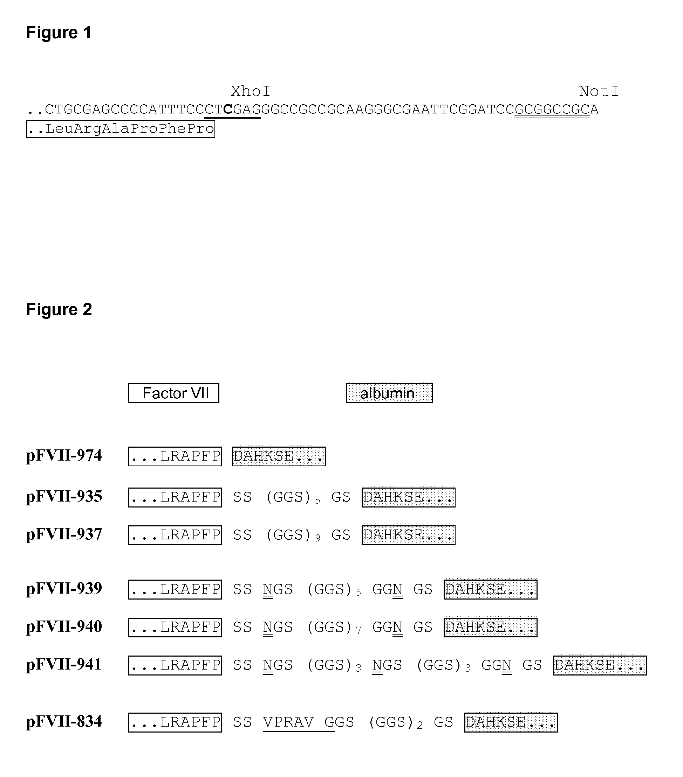

Subsequently an Xhol restriction site was introduced into pFVII-659 at the site of the natural FVII stop codon (figure 1) by site directed mutagenesis according to standard protocols (QuickChange XL Site Directed Mutagenesis Kit, Stratagene) using oligonucleotides We1643 and We 1644 (SEQ ID NO 5 and 6). The resulting plasmid was designated pFVII-700.

Oligonucleotides We1731 and We1732 (SEQ ID NO 7 and 8) were annealed in equimolar concentrations (10 pmol) under standard PCR conditions, filled up and amplified using a PCR protocol of a 2 min. initial denaturation at 94°C followed by 7 cycles of 15 sec. of denaturation at 94°C, 15 sec. of annealing at 55°C and 15 sec. of elongation at 72°C, and finalized by an extension step of 5 min at 72°C. The resulting fragment was digested with restriction endonucleases Xhol and Notl and ligated into pFVII-700 digested with the same enzymes. The resulting plasmid was designated pFVII-733, containing coding sequence for FVII and a C-terminal extension of a thrombin cleavable glycine/serine linker.

Based on pFVII-733 other linkers without thrombin cleavage site and additional N-glycosylation sites were inserted. For that primer pairs We2148 and We2149 (SEQ ID NO 9 and 10), We2148 and We2151 (SEQ ID NO 9 and 11), We2152 and We2154 (SEQ ID NO 12 and 13), We2152 and We2155 (SEQ ID NO 12 and 14) and We2156 and We2157 (SEQ ID NO 15 and 16), respectively, were annealed and amplified as described above. The respective PCR fragments were digested with restriction endonucleases Xhol and BamH1 and inserted into pFVII-733 digested with the same enzymes. Into the BamH1 site of the resulting plasmids as well as into that of pFVII-733 a BamH1 fragment containing the cDNA of mature human albumin was inserted. This fragment had been generated by PCR on an albumin cDNA sequence using primers We1862 and We1902 (SEQ ID NO 17 and 18) under standard conditions. The final plasmids were designated pFVII-935, pFVII-937, pFVII-939, pFVII-940, pFVII-941 and pFVII-834, respectively.

In order to generate a FVII albumin fusion protein without linker, deletion mutagenesis was applied as above upon plasmid pFVII-935 using primers We2181 and We2182 (SEQ ID NO 25 and 26). The resulting plasmid was designated pFVII-974. The linker sequences and the C-terminal FVII and N-terminal albumin sequences of these plasmids are outlined in figure 2.

### Example 2: Generation of cDNAs encoding FIX and FIX - albumin fusion proteins

Factor IX coding sequence was amplified by PCR from a human liver cDNA library (ProQuest, Invitrogen) using primers We1403 and We1404 (SEQ ID NO 27 and 28). After a second round of PCR using primers We1405 and We1406 (SEQ ID NO 29 and 30) the resulting fragment was cloned into pCR4TOPO (Invitrogen). From there the FIX cDNA was transferred as an EcoRI Fragment into the EcoRI site of expression vector pIRESpuro3 (BD Biosciences) wherein an internal Xhol site had been deleted previously. The resulting plasmid was designated pFIX-496 and was the expression vector for factor IX wild-type.

For the generation of albumin fusion constructs the FIX cDNA was reamplified by PCR under standard conditions using primers We2610 and We2611 (SEQ ID NO 31 and 32) deleting the stop codon and introducing an Xhol site instead. The resulting FIX fragment was digested with restriction endonucleases EcoRI and Xhol and ligated into an EcoRI / BamH1 digested pIRESpuro3 together with one Xhol / BamH1 digested linker fragment as described below.

Two different glycine / serine linker fragments were generated: Oligonucleotides We2148 and We2150 (SEQ ID NO 9 and 33) were annealed in equimolar concentrations (10 pmol) under standard PCR conditions, filled up and amplified using a PCR protocol of a 2 min. initial denaturation at 94°C followed by 7 cycles of 15 sec. of denaturation at 94°C, 15 sec. of annealing at 55°C and 15 sec. of elongation at 72°C, and finalized by an extension step of 5 min at 72°C. The same procedure was performed using oligonucleotides We2156 and We2157 (SEQ ID NO 15 and 16).

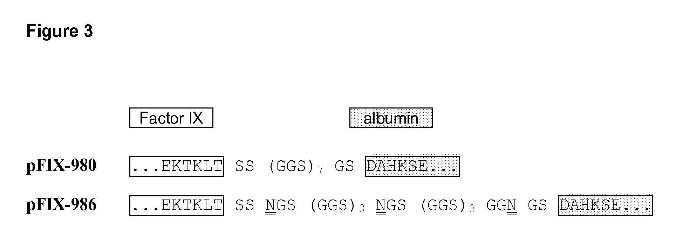

The resulting linker fragments were digested with restriction endonucleases Xhol and BamH1 and used separately in the above described ligation reaction. The resulting two plasmids therefore contained the coding sequence for FIX and a C-terminal extension of a glycine/serine linker. In the next cloning step these plasmids were digested with BamH1 and a BamH1 fragment containing the cDNA of mature human albumin was inserted. This fragment had been generated by PCR on an albumin cDNA sequence using primers We1862 and We1902 (SEQ ID NO 17 and 18) under standard conditions. The final plasmids were designated pFIX-980 and pFIX-986, respectively. Their linker sequences and the C-terminal FIX and N-terminal albumin sequences are outlined in figure 3.

For efficient processing of the propeptide in cells expressing FIX in high amounts coexpression of furin is required (Wasley LC et al. 1993. PACE/Furin can process the vitamin K-dependent pro-factor IX precursor within the scretory pathway. J. Biol. Chem. 268:8458-8465). Furin was amplified from a liver cDNA library (Ambion) using primers We1791 and We1792 (SEQ ID NO 34 and 35). A second round of PCR using primers We1808 and We1809 (SEQ ID NO 36 and 37) yielded a furin fragment where the carboxyterminal transmembrain domain (TM) was deleted and a stop codon introduced; this fragment was cloned into pCR4TOPO (Invitrogen). From there the furinΔTM cDNA was transferred as an EcoRI/NotI Fragment into the EcoRI/NotI sites of pIRESpuro3 (BD Biosciences) wherein an internal Xhol site had been deleted previously. The resulting plasmid was designated pFu-797. The amino acid sequence of the secreted furin encoded by pFu-797 is given as SEQ-ID NO 38.

### Example 3: Transfection and expression of FVII, FIX and respective albumin fusion proteins

Plasmids were grown up in E.coli TOP10 (Invitrogen) and purified using standard protocols (Qiagen). HEK-293 cells were transfected using the Lipofectamine 2000 reagent (Invitrogen) and grown up in serum-free medium (Invitrogen 293 Express) in the presence of 50 ng/ml Vitamin K and 4 µg/ml Puromycin. Cotransfection of furinΔTM cDNA was performed in a 1:5 (pFu-797: respective pFIX construct) molar ratio. Transfected cell populations were spread through T-flasks into roller bottles or small scale fermenters from which supernatants were harvested for purification.

### Example 4: Purification of FVII and FVII - albumin fusion polypeptides

Cell culture harvest containing FVII or FVII albumin fusion protein was applied on a 2.06 mL Q-sepharose FF column previously equilibrated with 20 mM Hepes buffer pH 7.4. Subsequently, the column was washed with 10 volumes of the named Hepes buffer. Elution of the bound FVII molecules was achieved by running a linear gradient from 0 to 1.0 M NaCl in 20 mM Hepes buffer within 20 column volumes. The eluate contained about 85-90% of the applied FVII antigen at protein concentrations between 0.5 and 1 g/L.
Alternatively FVII was purified by chromatography using immobilized tissue factor as described in EP 0770625B1.

FVII antigen and activity were determined as described in example 5.

### Example 5: Determination of FVII activity and antigen.

FVII activity was determined using a commercially available chromogenic test kit (Chromogenix Coaset FVII) based on the method described by Seligsohn et al. Blood (1978) 52:978-988.

FVlla activity was determined using a commercially available test kit (STACLOT®)VIIa-rTF, Diagnostica Stago) based on the method described by Morissey et al. (1993) Blood 81:734-744.

FVII antigen was determined by an ELISA whose performance is known to those skilled in the art. Briefly, microplates were incubated with 120 µL per well of the capture antibody (sheep anti human FVII IgG, Cedarlane CL20030AP, diluted 1:1000 in Buffer A [Sigma C3041]) overnight at ambient temperature. After washing plates three times with buffer B (Sigma P3563), each well was incubated with 200 µL buffer C (Sigma P3688) for one hour at ambient temperature. After another three wash steps with buffer B, serial dilutions of the test sample in buffer B as well as serial dilutions of standard human plasma (Dade Behring; 50 - 0.5 mU/mL) in buffer B (volumes per well: 100 µL) were incubated for two hours at ambient temperature. After three wash steps with buffer B, 100 µL of a 1:5000 dilution in buffer B of the detection antibody (sheep anti human FVII IgG, Cedarlane CL20030K, peroxidase labelled) were added to each well and incubated for another two hours at ambient temperature. After three wash steps with buffer B, 100 µL of substrate solution (TMB, Dade Behring, OUVF) were added per well and incubated for 30 minutes at ambient temperature in the dark. Addition of 100 µL undiluted stop solution (Dade Behring, OSFA) prepared the samples for reading in a suitable microplate reader at 450 nm wavelength. Concentrations of test samples were then calculated using the standard curve with standard human plasma as reference.

### Example 6: Purification of FIX and FIX - albumin fusion polypeptides

Cell culture harvest containing FIX or FIX albumin fusion protein was applied on a Q-sepharose FF column previously equilibrated with 50 mM TrisxHCl / 100 mM NaCl buffer pH 8.0. Subsequently, the column was washed with equilibration buffer containing 200 mM NaCl. Elution of the bound FIX or FIX fusion polypeptides was achieved by running a salt gradient. The eluate was further purified on hydroxylapatite by column chromatography. For this purpose, the eluate of the Q-Sepharose FF column was loaded on a hydroxylapatite chromatography column equilibrated with 50 mM TrisxHCl / 100 mM NaCl buffer pH 7.2. The column was washed with the same buffer and FIX or FIX-HAS were eluted using a phosphate salt gradient. The eluate was dialyzed to reduce the salt concentration and used for biochemical analysis as well as for determination the in vivo recovery. FIX antigen and activity were determined as described in example 7.

### Example 7: Determination of FIX antigen and activity.

FIX activity was determined as clotting activity using commercially available aPTT reagents (Dade Behring, Pathromtin SL and FIX depleted plasma).

FIX antigen was determined by an ELISA acc. to standard protocols known to those skilled in the art. Briefly, microplates were incubated with 100 µL per well of the capture antibody (Paired antibodies for FIX ELISA 1:200, Cedarlane) overnight at ambient temperature. After washing plates three times with blocking buffer B (Sigma P3563), each well was incubated with 200 µL buffer C (Sigma P3688) for one hour at ambient temperature. After another three wash steps with buffer B, serial dilutions of the test sample in buffer B as well as serial dilutions of a substandard (SHP) in buffer B (volumes per well: 100 µL) were incubated for two hours at ambient temperature. After three wash steps with buffer B, 100 µL of a 1:200 dilution in buffer B of the detection antibody (Paired antibodies for FIX ELISA, peroxidase labelled, 1:200, Cedarlane) were added to each well and incubated for another two hours at ambient temperature. After three wash steps with buffer B, 100 µL of substrate solution (TMB, Dade Behring, OUVF) were added per well and incubated for 30 minutes at ambient temperature in the dark. Addition of 100 µL undiluted stop solution (Dade Behring, OSFA) prepared the samples for reading in a suitable microplate reader at 450 nm wavelength. Concentrations of test samples were then calculated using the standard curve with standard human plasma as reference.

### Example 8: Comparison of FVII and FVII - albumin fusion proteins in respect to in vivo recovery

Recombinant FVII wild-type and FVII albumin fusion polypeptides described above were administered intravenously to narcotised CD / Lewis rats (6 rats per substance) with a dose of 100 µg/kg body weight. Blood samples were drawn at appropriate intervals starting at 5 minutes after application of the test substances from the arteria carotis. FVII antigen content was subsequently quantified by an Elisa assay specific for human Factor VII (see above). The mean values of the respective rat groups were used to calculate in vivo recovery.

The in vivo recovery was determined 5 min after application of the products (table 2). The FVII resp. FVIIa antigen levels measured per mL of plasma 5 min after intravenous application via the tail vein were related to the amount of product applied per kg. Alternatively, a percentage was calculated by relating the determined antigen level (IU/mL) 5 min post infusion to the theoretical product level expected at 100% recovery (product applied per kg divided by a theoretical plasma volume of 40 mL per kg).

The in vivo recoveries of the FVII fusion proteins determined accordingly in rats were found to be significantly increased in comparison to the non-fused recombinant wild type FVII. It was between 2.3 and 7.9 fold increased over wild type FVII depending on the construct used.

**Table 2: In vivo recovery of FVII and FVII - albumin fusion proteins In vivo recoveries (percentage of substance in circulation 5 minutes post application) of FVII wild-type and FVII albumin fusion proteins after intravenous application of 100µg/kg into rats (n= number of experiments).**

| **FVII polypeptide derived from pFVII** | **Albumin fusion** | **In vivo recovery [%]** | **Increase relative to wild-type (659) [%]** |
|---|---|---|---|
| 974 | yes | 56.7 | 787 |
| 935 | yes | 22.4 | 311 |
| 937 | yes | 45.6 (n=2) | 634 (n=2) |
| 939 | yes | 16.7 | 232 |
| 940 | yes | 31.3 (n=2) | 434 (n=2) |
| 941 | yes | 25.8 | 358 |
| 834 | yes | 27.3 (n=2) | 379 (n=2) |
| 659 | no | 7.2 (n=3) | 100 |
| (wild-type FVII) | | | |

### Example 9: Comparison of FIX and FIX - albumin fusion polypeptides in respect to in vivo recovery

Recombinant, commercially available FIX (BeneFIX, Wyeth, and rFIX wild-type) and FIX-albumin fusion polypeptides (rFIX-L-HSA 980/797 and rFIX-L-HSA 986/797) described above were administered intravenously to narcotised rabbits (4 rabbits per substance)and CD / Lewis rats (6 rats per substance), respectively, with a dose of 50 IU/kg body weight. Blood samples were drawn at appropriate intervals starting at 5 minutes after application of the test substances from the arteria carotis. FIX antigen content was subsequently quantified by an Elisa assay specific for human Factor IX (see above). The mean values of the respective groups were used to calculate in vivo recovery after 5 min.

Calculated in vivo recoveries 5 min post-infusion are summarized in table 3. The FIX antigen levels measured per mL of plasma 5 min after intravenous application via the tail vein were related to the amount of product applied per kg. Alternatively, a percentage was calculated by relating the determined antigen level (IU/mL) 5 min post infusion to the theoretical product level expected at 100% recovery (product applied per kg divided by an assumed plasma volume of 40 mL per kg).

In rats as well as in rabbits the in vivo recoveries of the FIX fusion proteins surprisingly were found to be significantly increased in comparison to the non-fused recombinant F IX prepared in house or the commercially available FIX product BeneFIX. The increase over BeneFIX was 49.7, 69.4 or 87.5%, depending on the animal species or construct used. Compared to the corresponding wild type FIX, the recovery increases of the FIX fusion proteins were even higher.

**Table 3:**

| In vivo recoveries (amount of substance 5 minutes post application) of recombinant FIX preparations (BeneFIX, rFIX 496/797) and FIX albumin fusion proteins (rFIX-L-HSA 980/797 and rFIX-L-HSA 986/797) after intravenous application of 50 IU /kg into rats and 50 IU /kg into rabbits, respectively. The percentage of in vivo recovery was calculated based on an assumed plasma volume of 40 mL/kg). | | | | |
|---|---|---|---|---|
| | rat experiment | | rabbit experiment | |
| | | | | |
| | in vivo recovery IU/mL per IU/kg / [%]* | relative to BeneFIX [%] | In vivo recovery IU/mL per IU/kg / [%]* | relative to BeneFIX [%] |
| rFIX 496/797 | 0.462/18.5 | 74.6 | n.d.** | - |
| rFIX-L-HSA | 1.162/46.5 | 187.5 | 1.26 / 50.6 | 149.7 |
| 980/797 | | | | |
| rFIX-L-HSA | 1.051/42.0 | 169.4 | n.d.** | - |
| 986/797 | | | | |
| BeneFIX | 0.621 /24.8 | 100 | 0.846/33.8 | 100 |
| | | | | |

| | | | | |
|---|---|---|---|---|
| * Calculated based on a plasma volume of 40 mL/kg ** not determined | | | | |

## Claims

1. Use of a therapeutic polypeptide fused directly or via a linker peptide to a polypeptide enhancing the in-vivo recovery as compared to the in-vivo recovery of the corresponding non-fused therapeutic polypeptide and wherein the recovery enhancing polypeptide is albumin, a naturally occurring polymorphic variant or a fragment thereof, wherein the fragment is at least 20 amino acids long, for increasing the in vivo recovery.

2. Use of a therapeutic polypeptide according to claim 1 for increasing the in vivo recovery wherein the therapeutic polypeptide is formulated for subcutaneous administration.

3. Use according to claims 1 or 2, wherein the therapeutic polypeptide comprises a vitamin K-dependent protein.

4. Use according to claims 1 to 3, wherein the therapeutic polypeptide comprises Factor IX, Factor VII or Factor VIIa or a fragment or variant thereof.

5. Use according to claim 3 or 4, wherein the therapeutic polypeptide moiety is fused to the N-terminus of the albumin moiety.

6. Use according to claim 5, wherein the therapeutic polypeptide is Factor VII or Factor VIIa.

7. Use according to claim 6, **characterized in that** a peptidic linker separates the Factor VII or Factor VIIa moiety from the albumin moiety.

8. Use according to claim 7, **characterized in that** the peptidic linker is modified by the insertion of sites for posttranslational modifications.

9. Use according to claim 8, **characterized in that** the posttranslational modification comprises one or more N-glycosylation sites of the structure Asn - X - Ser/Thr, wherein X denotes any amino acid except proline.

10. Use according to claims 6 to 9, **characterized in that** the Factor VII polypeptide moiety has procoagulant activity.

11. Use according to claim 5, wherein the therapeutic polypeptide is Factor IX.

12. Use according to claim 11, **characterized in that** a peptidic linker separates the Factor IX moiety from the albumin moiety.

13. Use according to claim 12, **characterized in that** the peptidic linker is modified by the insertion of sites for posttranslational modifications.

14. Use according to claim 13, **characterized in that** the posttranslational modification comprises one or more N-glycosylation sites of the structure Asn - X - Ser/Thr, wherein X denotes any amino acid except proline.

15. Use according to claims 11 to 14, **characterized in that** the Factor IX polypeptide moiety has procoagulant activity.
